# EUROPÄISCHE PATENTSCHRIFT

(11) **EP 3 214 447 B1**
(45) Veröffentlichungstag und Bekanntmachung des Hinweises auf die Patenterteilung: **26.09.2018**
(21) Anmeldenummer: 17158291.9
(22) Anmeldetag: 28.02.2017
(51) Int. Cl.: G01N 33/84, G01N 21/80

(54) **TEST ZUR BESTIMMUNG EINER BASEN-KONZENTRATION**
TEST FOR THE DETERMINATION OF A BASE CONCENTRATION
TEST DESTINÉ À DÉTERMINER UNE CONCENTRATION DE BASES

(30) Priorität: 01.03.2016 DE 102016203335
(43) Veröffentlichungstag der Anmeldung: 06.09.2017
(73) Patentinhaber: AXAGARIUS GmbH & Co. KG, 52355 Düren (DE)
(72) Erfinder: Lange, Dominik, 50733 Köln (DE); Hoffmann, Jürgen, 52355 Düren (DE)
(74) Vertreter: Jostarndt Patentanwalts-AG

(56) Entgegenhaltungen:
- EP-A1- 1 548 443
- US-B2- 6 986 999

## Beschreibung

Die vorliegende Erfindung betrifft eine Vorrichtung zur Bestimmung der Konzentration einer Base, und hierbei insbesondere Hydrogencarbonat, wobei die Vorrichtung einen pH-Indikator, eine feststoffliche Säure und ein Gemisch aus einem anionischen und einem nichtionischen Tensid enthält. Die Erfindung betrifft zudem ein Verfahren zur Messung der Basen-Konzentration unter Verwendung der erfindungsgemäßen Vorrichtung und ein Verfahren zur Sensitivitätserhöhung einer Testvorrichtung.

Dialysate werden im Allgemeinen mit einer sauren (z.B. Citronensäure, Puffersalze, Glucose) und einer basischen (z.B. Hydrogencarbonat, Acetat, Lactat) Komponente sowie Wasser (Osmosewasser) angesetzt. Im Gegensatz zu den etablierten stabilen Puffersubstanzen Acetat und Lactat ist die Verwendung von Hydrogencarbonat technisch problematisch, da die Stabilität in Lösungen nicht gegeben ist. Der große Vorteil von Hydrogencarbonat als Puffersubstanz ist die hohe physiologische Verträglichkeit und die damit verbundene geringere Wahrscheinlichkeit von Nebenwirkungen. Gewöhnlich erfolgt die Ansatzkontrolle der Dialysate über die Bestimmung des pH-Werts mit Hilfe eines pH-Teststreifens.

Die zusätzliche Bestimmung der Hydrogencarbonat-Konzentration erhöht weiter die Sicherheit für den Patienten, da falsche Hydrogencarbonat-Konzentrationen im Dialysat zu pathologischen Veränderungen des Blut-Puffersystems des Patienten führen. So kann ein hyperosmolares (zu stark konzentriertes) Dialysat zu Hypernatriämie und anderen elektrolytischen Störungen führen, wohingegen ein hypoosmolares (also zu stark verdünntes) Dialysat eine rasche Hämolyse oder einer Hyponitriämie auslösen kann. Es ist daher von entscheidender Bedeutung, die korrekte Hydrogencarbonat-Konzentration im Dialysat zu bestimmen.

Vorrichtungen und Testverfahren zur Bestimmung der Dialysatzusammensetzung sind im Stand der Technik bekannt. Im Allgemeinen wird hierbei die Konzentration der ionischen Komponenten des Dialysats indirekt durch die elektrische Leitfähigkeit des Dialysats festgestellt, basierend auf der Tatsache, dass hauptsächliche Dialysatbestandteile Elektrolyte sind. Zudem wird auch der pH-Wert des Dialysats erfasst.

Nachteilig ist hierbei, dass die dazu verwendeten Gerätschaften wie Leitfähigkeitsmessgeräte und Glas-pH-Elektroden Routinewartungen und eine regelmäßige Kalibrierung benötigen, um eine adäquate Funktion zu gewährleisten.

Die Leitfähigkeitsmessung weist beachtlicherweise einen gravierenden verfahrensinhärenten Nachteil auf, insofern sie lediglich eine summarische Darstellung der in der Lösung enthaltenden lonenkomponenten leistet. So kann bei einer korrekten Leitfähigkeit ein falsches Verhältnis von Hydrogencarbonat und Säure vorliegen.

Die pH-Wertmessung weist zudem als logarithmisches Meßverfahren eine allzu geringe Sensitivität auf, insofern nur sehr große Veränderungen des Verhältnisses von Hydrogencarbonat und Säure als pH-Wertänderungen erfaßbar sind.

Als Alternativmethode zur Bestimmung der Hydogencarbonat-Konzentration schlägt die i US 6,986,999 B2 (Serim Research Corp.) die Verwendung von Teststreifen vor, bei denen eine Trägermatrix eine organische Säure, einen pH-Indikator und einen Inertfarbstoff enthält und nach Eintauchen in das Dialysat der Farbumschlag mit einem Farbstandard verglichen wird. Diese Teststreifen decken einen physiologisch irrelevant großen Messbereich von 18.5 bis 74 mEq/L ab und erlauben hierbei lediglich eine Unterscheidung von 18.5 vs. 37 vs. 74 mEq/L. Sie sind damit nur unzureichend für eine sensitive und damit aussagekräftige Bestimmung von Dialysaten geeignet.

Die EP 1 548 443 A1 betrifft einen Prüfkörper zur Bestimmung eines sauren Proteins, wobei der Prüfkörper einen sauren pH-Indikator umfasst und durch die zusätzliche i Verwendung eines Tensids im Prüfkörper die Farbsensitivität des pH-Indikators erhöht wird. Der Prüfkörper der EP'443 ist nicht für die Bestimmung von Hydrogencarbonat geeignet, da er selber keine organische Säure enthält und lehrt zudem lediglich die Kombination eines nichtionischen und eines kationischen Tensids.

Es besteht daher ein Bedarf an verbesserten analytischen Verfahren und Vorrichtungen zur Bestimmung der Hydrogencarbonat-Konzentration.

Aufgabe der Erfindung ist es daher, eine gattungsgemäße Testvorrichtung bzw. ein Testverfahren zur Bestimmung der Hydrogencarbonat-Konzentration dahingehend zu i verbessern, dass sie bezüglich mindestens einer der oben genannten Nachteile verbessert wird.

### Zusammenfassung der Erfindung

Diese Aufgabe wird gemäß der Erfindung dadurch gelöst, dass eine Testvorrichtung zur Bestimmung der Konzentration einer Base bereitgestellt wird, wobei die Testvorrichtung eine Trägermatrix enthält, die folgendes umfasst:
a) mindestens eine Säure, die bei Raumtemperatur fest ist;
b) mindestens einen pH Indikator mit einem Umschlagspunkt zwischen dem pH-Wert der feststofflichen Säure und der zu bestimmenden Base; und
c) ein Gemisch aus mindestens einem anionischen und mindestens einem nichtionischen Tensid.

Die erfindungsgemäße Testvorrichtung vereinigt mehrere entscheidende Vorteile gegenüber den aus dem Stand der Technik bekannten Testvorrichtungen.

Wie die Erfinder festgestellt haben führt die Anwesenheit eines Gemisches aus anionischen und nichtionischen Tensid zu einer signifikanten Erhöhung der Sensitivität des Messverfahrens. So können Hydrogenkarbonat-Konzentrationen, die sich nur in 5 mEq/L-Einheiten unterscheiden (also bspw. 25; 30; 35, 40 und 45 mEq/L) in einfacher Weise bestimmt werden. Damit werden erstmalig auch klinisch relevante Änderungen in der Hydrogencarbonat-Konzentration in eindeutiger Weise detektierbar.

Zudem erlaubt der Tensidgemisch-Zusatz eine Einengung des Messbereichs auf den physiologisch/klinisch relevanten Messbereich von 25 bis 45 mEq/L Hydrogencarbonat.

Die daraus resultierende Messung stellt eine entscheidende Verbesserung in der Dialysat-Analytik dar, da sie erstmalig und auf einfache und direkte Weise eine hochsensitive Messung der Dialysatzusammensetzung möglich macht. Dies ist durch die Tatsache möglich, dass die weiteren Dialysatbestandteile, in ihrer üblichen Konzentration, das erfindungsgemäße Testverfahren nicht stören.

Da die Testvorrichtung auf einer schnellen Säure-Base-Reaktion mit direktem Farbnachweis beruht stellt sie ein direktes und sehr schnelles Nachweisverfahren dar. Zudem ist die erfindungsgemäße Testvorrichtung einfach zu gebrauchen und zu interpretieren und bedarf keiner zusätzlichen Meßgeräte. Gerade im klinischen Alltag erhöht dies die Compliance, da auch ein Benutzer ohne technische oder medizinische Ausbildung einen raschen und zuverlässigen Sichtnachweis durchführen kann.

Die Testvorrichtung erlaubt eine selektive, d.h. lediglich auf basische Substanzen ausgerichtete Bestimmung.

Die Testvorrichtung weist auch eine ausreichende Stabilität auf und bedarf bei Verwendung der herkömmlichen Reagenzien auch keiner Kühlung.

Durch die Auswahl geeigneter Inhaltsstoffe erlaubt die Robustheit der Testvorrichtung eine bestimmte Variabilität der zu analysierenden Probe (z.B. Probenvorbehandlung nicht erforderlich) und/oder anderer definierter physikalischer Parameter während des Messvorganges und liefert trotzdem reproduzierbare und standardisierbare Ergebnisse.

Sie erfüllt zudem somit alle Anforderungen, die eine klinisch eingesetzte Testvorrichtung bzw. ein Testverfahren erfüllen müssen, und ist somit auch validierbar.

Die Testvorrichtung kann mit handelsüblichen Substanzen auf einfache Weise und zudem kostengünstig hergestellt werden.

Die Testvorrichtung kann ohne Mehraufwand in die bereits etablierten Testsysteme integriert werden.

Der Fachmann kann hinsichtlich der einzelnen Komponenten auf eine Vielzahl an chemischen Substanzen zurückgreifen und die Vorrichtung so gezielt an die jeweils zu bestimmende Base anpassen. Insbesondere die Auswahl der feststofflichen Säure und des pH-Indikators erlauben eine Anwendung auf verschiedene Basen.

Weiterhin kann er durch eine gezielte Veränderung der Menge an Nachweisreagenzien den Konzentrations-Messbereich einstellen.

### Ausführliche Beschreibung der Erfindung

Die Testvorrichtung basiert auf einer Titration der vorhandenen Säure mit der zu bestimmenden Base. Der pH-Indikator, der mit seinem Umschlagpunkt oberhalb des Säure-pH-Wertes aber unterhalb des pH-Wertes der zu bestimmenden Base liegt, vermag den durch die Säure-Base-Reaktion resultierenden Anstieg des pH-Wertes durch einen entsprechenden Farbumschlag anzuzeigen. Die Endfarbe des Indikators kann mit unterschiedlichen Methoden ausgewertet werden. In der einfachsten Form wird sie mit einer entsprechenden Farbe auf einer Standardfarbkarte verglichen, bei der die einzelnen Farbwerte bestimmten Basenkonzentrationen oder entsprechend bestimmten pH-Werten zugeordnet sind.

Da die Testvorrichtung auf der Hochtitration der vorgegebenen feststofflichen Säure basiert, kann sie zur Bestimmung aller denkbaren Basen eingesetzt werden und somit für alle alkalisch reagierenden Verbindungen eingesetzt werden.

So können neben neutralen Basen wie NH₃ oder Amine anionische Basen wie das Hydrogencarbonat-Anion, kationische Basen wie das [Al³⁺(OH)⁻(H₂O)₅], einwertige Basen wie Natriumhydroxid oder Kaliumhydroxid, zweiwertige Basen wie das Calciumhydroxid oder Basenbildner wie Calciumoxid, Bariumoxid oder Alkalimetalle mit Hilfe der Testvorrichtung in ihrer Konzentration bestimmt werden.

In bevorzugter Weise wird die Testvorrichtung zur Konzentrationsbestimmung von Hydrogencarbonat eingesetzt.

Bei der Anwendung zur Hydrogencarbonat-Bestimmung erlaubt die Testvorrichtung die Messung in einem engen Messbereich von 25-45 mEq/L Hydrogencarbonat und auch eine feinere Abstufung der Farbreaktion mit Skalenwerten, die sich nur um 5 mEq/L unterscheiden und bevorzugt 25, 30, 35, 40 und 45 mEq/L Hydrogencarbonat entsprechen.

Beachtlicherweise werden im klinischen Alltag die Dialysate von Dialysemaschinen hergestellt, die durch eine gesteuerte Zufuhr eines Dialyse-Konzentrats ein Dialysat mit einer Zielkonzentration von 37±2 mmol/l (mEq/L) Hydrogencarbonat herstellen. Das vorliegende Messverfahren ist also optimal auf diese Hydrogencarbonat-Konzentration abgestimmt. Unter Berücksichtigung der maschinellen Parameter, kann bei den Dialysemaschinen aus dem Stand der Technik mit abweichenden Hydrogencarbonat-Konzentrationen in einem Bereich von ca. 25 bis ca. 45 mEq/L gerechnet werden, also genau der Bereich der von der erfindungsgemäßen Testvorrichtung abgedeckt wird.

In einer weiteren Ausführungsform sieht die Testvorrichtung die Verwendung mindestens eines Inertfarbstoffs vor. Dieser Inertfarbstoff ist auf den pH-Indikator und dessen Farbumschlag abgestimmt und erlaubt durch eine gleichbleibende Hintergrundfärbung eine bessere Detektion des Farbumschlags. So ist bei einem von Blau nach Grün umschlagenden pH-Indikators die Verwendung eines gelben Inertfarbstoffs vorteilhaft.

Der Inertfarbstoff ist erfindungsgemäß im gleichen Abschnitt wie der pH-Indikator bereitzustellen. Dies kann in mehrfacher Hinsicht realisiert werden, wie bspw.:
(a) Der Inertfarbstoff kann auf dem Träger selber aufgebracht sein, bzw. durch einen gefärbten Träger bereitgestellt werden.
(b) Der Inertfarbstoff kann mit in die Trägermatrix eingebracht werden.
(c) Die Trägermatrix selber kann farbig ausgestaltet sein.
(d) Die Testvorrichtung kann unter der Trägermatrix noch eine separate gefärbte Schicht aufweisen.

In bevorzugter Weise wird genau ein Inertfarbstoff eingesetzt. Es können allerdings auch zwei, drei oder noch mehr Inertfarbstoffe eingesetzt werden, um hier eine für das System optimale Farbigkeit zu erzielen.

In besonders bevorzugter Weise enthält hierbei die Trägermatrix genau einen Inertfarbstoff.

In einer Ausführungsform der Erfindung ist der mindestens eine Inertfarbstoff ausgewählt aus der Gruppe enthaltend Tartrazin, Neozapongelb und Nitrazingelb, wobei der Inertfarbstoff bevorzugt Nitrazingelb ist.

Die feststoffliche Säure der Testvorrichtung dient der Reaktion mit der zu bestimmenden Base und erlaubt durch die pH-Wertänderung die Quantifizierung derselben. Insofern sie als Feststoff vorliegt, verflüchtigt sie sich nicht, sondern verbleibt als Feststoff in der Trägermatrix und erlaubt eine langzeitstabile Formulierung.

Zweckmäßigerweise handelt es sich bei der Säure um eine in der zu bestimmenden flüssigen Lösung gut lösliche Säure, so dass die zugegebene Testlösung die feste Säure sofort auflöst und damit eine effiziente Säure-Base-Reaktion ermöglicht. Da die meisten Testlösungen wässrige Testlösungen darstellen, sind somit gut wasserlösliche Säuren, wie organische Säuren bevorzugt.

Bei der Bestimmung eines basischen Ampholyten, wie es beispielsweise das Hydrogencarbonatanion darstellt, sollte die Säure einen pKa-Wert aufweisen, der etwa eine Einheit unter dem pKa-Wert des Ampholyten liegt.

In einer bevorzugten Ausführungsform handelt es sich bei der Säure um eine physiologisch unbedenkliche Substanz.

In einer Ausführungsform der Erfindung ist die mindestens eine feststoffliche Säure ausgewählt ist aus der Gruppe enthaltend Zitronensäure, Bernsteinsäure, Weinsäure, Phthalsäure, Fumarsäure, Gluconsäure, Äpfelsäure, Glykolsäure, Malonsäure, Glutarsäure, Adipinsäure, Ascorbinsäure, Amidoschwefelsäure, Borsäure, Diphosphorsäure und Phosphonsäure.

In besonders bevorzugter Weise handelt es sich bei der Säure um Weinsäure.

Für die Bestimmung schwacher Basen mit einem pK_{B}-Wert von 7.5 bis 9, wie beispielsweise für Hydrogencarbonat, ist es bevorzugt, dass die mindestens eine Säure einen pKs-Wert zwischen 2,9 und 5,6 aufweist

In bevorzugter Weise wird genau eine Säure eingesetzt. Es können allerdings auch zwei, drei oder noch mehr Säuren eingesetzt werden, um bspw. eine mehrwertige Base mit unterschiedlichen pK_{B}-Werten optimal zu erfassen.

Erfindungsgemäß setzt die Testvorrichtung die Anwesenheit mindestens einen pH-Indikators voraus.

Für die Bestimmung schwacher Basen mit einem pK_{B}-Wert von 7.5 bis 9, ist es bevorzugt, dass der mindestens eine pH-Indikator einen Umschlagspunkt zwischen von zwischen pH=3,8 und pH=7,6 aufweist. In besonders bevorzugter Weise wird hierbei Bromphenolblau verwendet.

Für die Bestimmung eines Ampholyten wie Hydrogencarbonat mit einem pKs-Wert von 10,4 und einem pK_{B}-Wert von 7,5, ist es bevorzugt, dass der mindestens eine pH-Indikator einen Umschlagspunkt von weniger als pH=4,6, und bevorzugt von zwischen 3,0 und 4,6 aufweist. In besonders bevorzugter Weise wird hierbei Bromphenolblau verwendet.

In einer weiteren Ausführungsform der Erfindung ist der mindestens eine pH-Indikator ausgewählt aus der Gruppe enthaltend Bromphenolblau, Methylorange, Tetrabromphenolblau, Kongorot, Bromkresolgrün, Lackmus und Phenolrot.

Es können je nach vorliegender Säure und zu bestimmender Base auch gänzlich andere pH-Indikatoren eingesetzt werden und der Fachmann kann diese anhand der vorliegenden pH, pKs und pK_{B}-Werte gezielt aus der breiten Palette der verfügbaren Indikatoren auswählen.

In bevorzugter Weise wird genau ein pH-indikator eingesetzt. Es können allerdings auch zwei, drei oder noch mehr pH-Indikatoren eingesetzt werden, um bspw. den pH-Wertmessbereich noch breiter zu gestalten.

Die erfindungsgemäße Mischung verschiedener Tensidklassen ist entscheidend für die verbesserte Analytik des vorliegenden Messverfahrens und beruht auf der Mischung von nichtionischen und anionischen Tensiden.

In einer Ausführungsform ist das mindestens eine nichtionische Tensid ausgewählt aus der Gruppe enthaltend Fettalakoholethoxylate (FAEO) wie Brij35, Fettalakoholpropoxylate (FAPO), Alkylglucoside wie Tween20, Alkylpolyglucoside (APG), Oktylphenolethoxylate und Nonidet P40

In einer bevorzugten Ausführungsform ist das nichtionische Tensid Nonidet P40.

In einer Ausführungsform ist das mindestens eine anionische Tensid ausgewählt aus der Gruppe enthaltend Natriumdodecylsulfat, Ammoniumdodecylsulfat, Natriumlaurylethersulfat (SLES), Natriummyristylethersulfat, Natriumdioctylsulfosuccinat, Perfluoroctansulfonat (PFOS), Perfluorbutansulfonat und lineare Alkylbenzolsulfonate.

In einer bevorzugten Ausführungsform ist das anionische Tensid Natriumdodecylsulfat.

In einer weiteren Ausführungsform der Erfindung liegt das molare Verhältnis zwischen dem anionischen und nichtionischen Tensid zwischen 10:1 und 1:1, bevorzugt zwischen 5:1 und 1:1, besonders bevorzugt zwischen 4:1 bzw. 3:1 und 1:1 und insbesondere bevorzugt zwischen 2:1 und 1:1.

In spezieller Weise liegt das molare Verhältnis hierbei zwischen 2:1 und 1,5:1 und ganz speziell bei 1,6:1.

In einer Ausführungsform liegt ein Nachweisreagenz, oder mehrere Nachweisreagenzien oder sogar alle Nachweisreagenzien als in der Trägermatrix immobilisiert vor.

In einer Ausführungsform der Erfindung ist die Testvorrichtung als Teststreifen oder Testband ausgebildet oder so ausgestaltet, dass sie in ein integriertes Testsystem aufnehmbar ist.

Der Teststreifen kann aus einer Vielzahl von Materialien hergestellt werden. Bevorzugt sind hierbei wasserfeste Materialien wie Kunststoffe. Der Teststreifen besteht hierbei bevorzugt aus Polyvinylchlorid oder Polyethylen.

In einer weiteren Ausführungsform kann die Testvorrichtung weitere Trägermatrizes aufweisen.

Durch eine oder mehrere zusätzliche Trägermatrizes, die ebenfalls zur Bestimmung der Basenkonzentration ausgerichtet sind, können unterschiedliche Messbereiche abgedeckt werden.

Zweckmäßigerweise können die zusätzlichen Trägermatrizes auch zur Bestimmung anderer Zielanalyten ausgestaltet sein.

So kann die für die Dialysatanalyse verwendbare Testvorrichtung neben einer Trägermatrix zur Hydrogencarbonat-Bestimmung auch eine Trägermatrix zur Bestimmung des Glucosegehalts und/oder des pH-Wertes enthalten. Entsprechende Trägermatrizes und Nachweisreagenzien sind dem Fachmann bekannt.

So kann die Bestimmung des pH-Werts über einen pH-Indikator erfolgen und die Bestimmung der Glucose durch eine Oxidation unter Verwendung des Enzyms Glucoseoxidase (GOD; EC 1.1.3.4) unter Bildung von Wasserstoffperoxid. Das Wasserstoffperoxid wird dann in einer nachgeschalteten Farbreaktion zu Wasser reduziert. Diese Farbreaktion wird durch eine Peroxidase (POD) - meist Meerrettichperoxidase (EC 1.11.1.7) - katalysiert. Entsprechend wird das Testsystem auch als "GOD/POD-Test" bezeichnet.

Die erfindungsgemäße Trägermatrix ist zweckmäßigerweise aus einem Material, dass den Durchtritt von Flüssigkeiten erlaubt. Erfindungsgemäß handelt es sich hierbei insbesondere um poröse Materialien, die bevorzugterweise die Flüssigkeit aufsaugen und so eine definierte Menge an Flüssigkeit in Reaktion mit den Nachweisreagenzien bringen.

Dem Fachmann sind aus dem Stand der Technik zahlreiche Materialien und Strukturen bekannt die als Trägermatrix geeignet sind und er kann in Abhängigkeit vom Testansatz diese gezielt auswählen.

In einer Ausführungsform der Erfindung ist die Trägermatrix ausgewählt ist aus der Gruppe enthaltend Filterpapier, Vliesstoffe, Glasfaser, poröses Polymermaterial aus Polysulfon, Polyester, Nylon, Nitrocellulose, PVDF und Polycarbonat.

In einer bevorzugten Ausführungsform handelt es sich bei der Trägermatrix um ein Filterpapier. Filterpapiere sind kostengünstig und stark saugfähig und können in einfacher Weise (durch Tränken und anschließendes Trocknen) mit den Testreagenzien versehen werden.

In einer Ausführungsform ist die Trägermatrix einschichtig aufgebaut, so dass alle Nachweisreagenzien in dieser Schicht enthalten sind. In einer alternativen Ausführungsform kann die Trägermatrix aus zwei oder mehr Schichten aufgebaut sein. Die einzelnen Schichten können so beispielsweise eine unterschiedliche Saugfähigkeit bzw. Aufnahmekapazität für Flüssigkeiten aufweisen, so dass die flüssige Probe gezielter aufgenommen werden kann und auch ein Ausbluten der Trägermatrix verhindert werden kann. Zudem erlaubt dies eine räumliche Trennung der unterschiedlichen Nachweisreagenzien, so dass chemisch/physikalisch nicht-kompatible Nachweisreagenzien verwendet werden können, oder die von außen eindringende flüssige Probe beim Durchdringen der einzelnen Schichten sequentiell mit den Nachweisreagenzien reagieren kann.

Weiterhin kann die Trägermatrix auch einen als "Waste-Pad" ("Abfall-Pad") bezeichneten Bereich aufweisen, der die durch die Trägermatrix durchgelaufene Flüssigkeit aufnimmt. In diesem Bereich kann z.B. eine saugfähige Matte, bzw. ein Vlies, ein Lösch- oder Filterpapier oder dergleichen vorgesehen sein.

Die Ausgestaltung der Trägermatrix kann in Form und Tiefe derart erfolgen, dass eine kleine Chromatographiesäule entsteht, bei der evtl. störende Probenbestandteile abgetrennt werden können.

In einer besonderen Ausführungsform, stellt die Erfindung eine Testvorrichtung zur Bestimmung der Hydrogencarbonat-Konzentration bereit, wobei die Trägermatrix folgendes umfasst:
a) Weinsäure
b) Nitrazingelb
c) Bromphenolblau
d) Natriumdodecylsulfat
e) Nonidet P40

In einer besonders bevorzugten Ausführungsform ist die vorgenannte Trägermatrix auf einem Teststreifen aufgebracht.

In einem zweiten Aspekt stellt die Erfindung ein Testverfahren zur Bestimmung der Hydrogencarbonat-Konzentration in einer flüssigen Probe unter Verwendung der erfindungsgemäßen Testvorrichtung bereit, dass die folgenden Schritte umfasst:
a) Tränken der Trägermatrix mit der flüssigen Probe, bevorzugt durch Eintauchen in die Probe;
b) Entfernen überschüssigen Probenmaterials von der Trägermatrix, bevorzugt durch Herausziehen des Teststreifens aus der Probe;
c) Optional eine Inkubation des Teststreifens für mindestens 5 Sekunden, bevorzugt bei Raumtemperatur;
d) Vergleich des Farbwertes der Trägermatrix mit einem Farbstandard,

In einer bevorzugten Ausführungsform ist die flüssige Probe ein Dialysat.

In einem dritten Aspekt betrifft die Erfindung die Verwendung eines Gemischs aus mindestens einem anionischen Tensid und mindestens einem nichtionischen Tensid zur Steigerung der Sensitivität eines Trägermatrix-vermittelten Analyseverfahrens.

Hierbei ist es bevorzugt, wenn die vorab offenbarten anionischen und nichtionischen Tenside und die entsprechenden molaren Verhältnisse eingesetzt werden.

In einer bevorzugten Ausführungsform handelt es sich bei diesem Trägermatrix-vermittelten Analyseverfahren, um ein Verfahren, dass auf einer Säure-Base-Reaktion basiert und in besonders bevorzugter Weise eine Trägermatrix betrifft, die mindestens eine Säure, mindestens einen pH-Indikator und gegebenenfalls einen Inertfarbstoff umfasst.

### Definitionen

Unter einer "Testvorrichtung" werden im Rahmen der vorliegenden Erfindung alle trägergebundenen Tests für medizinische und nichtmedizinische Zwecke verstanden. Bei diesen trägergebundenen Tests sind Nachweisreagenzien in der Trägermatrix eines Trägers eingebettet, der mit der flüssigen Probe in Kontakt gebracht wird. Die Reaktion von flüssiger Probe und Reagenzien führt bei Anwesenheit des Zielanalyten, also hier der Base, zu einem nachweisbaren Signal, beispielsweise einem messbaren elektrischen Signal oder einem Farbumschlag, welcher visuell oder mit Hilfe eines Geräts beispielsweise transmissionsphotometrisch, reflexionsphotometrisch oder fluoreszenzphotometrisch ausgewertet werden kann.

Unter "Basen" sind erfindungsgemäß alle chemischen Substanzen zu verstehen, die bei Lösung in Wasser bei 25°C einen pH-Wert von >7.0 ergeben. Dies können neben neutralen Basen wie NH₃, anionische Basen wie das Hydrogencarbonat-Anion, kationische Basen wie das [Al³⁺(OH)⁻(H₂O)₅], einwertige Basen wie Natriumhydroxid oder Kaliumhydroxid, zweiwertige Basen wie das Calciumhydroxid oder Basenbildner wie Calciumoxid, Bariumoxid oder Alkalimetalle sein.

Gemäß der Erfindung ist unter einer "feststofflichen Säure" eine Säure zu verstehen, die bei Raumtemperatur in einem festen Aggregatzustand vorliegt. Dies kann sowohl eine organische Säure als auch eine anorganische Säure sein.

Im Rahmen der Erfindung ist unter einem "pH-Indikator" eine Substanz zu verstehen, die in Abhängigkeit von dem pH-Wert ihre Farbe ändert.

Erfindungsgemäß ist unter einem "Inertfarbstoff" ein Farbstoff zu verstehen, der zumindest im testgemäßen pH-Bereich (also zwischen dem pH-Wert der vorgelegten Säure und dem pH-Wert der zu bestimmenden Base) seine Farbe nicht oder nur unwesentlich ändert und somit einen gleichbleibenden farbkontrastierenden Hintergrund ermöglicht.

Unter "Selektivität" versteht man die Fähigkeit bestimmter Substanzen, aus einer Anzahl gebotener Möglichkeiten zur Reaktion eine bevorzugt auszusuchen. Die ausschließliche Auswahl bezeichnet man als Spezifität.

Als "Sensitivität" (Empfindlichkeit) bezeichnet man die Stärke der Änderung in der Antwort eines Messsignals geteilt durch die Änderung der auslösenden Größe (z.B. der Zielanalytkonzentration). Die Empfindlichkeit einer analytischen Methode entspricht der Steigung der Kalibrierkurve.

Erfindungsgemäß sind die zu unterscheidenden Begriffe der "Systempräzision" (Messpräzision) und der "Methodenpräzision" wie folgt definiert: Die Messpräzision ist ein Maß für die Schwankungen, die durch die Testvorrichtung oder dem damit arbeitenden Analysengerät selbst verursacht werden. Sie wird durch die Mehrfachanalyse (z. B. sechsfach) eines Standards ermittelt. Die Forderung an die Messpräzision hängt vom Analysengerät ab. Dagegen beschreibt die Methodenpräzision die zufällige Streuung der Analysenergebnisse. Sie wird durch eine mehrfache (meist sechsfache) Durchführung der gesamten Analyse, d. h. vom Abwiegen über die Probenvorbereitung bis zu der Messung und Befund ermittelt (sechs Einwaagen realer Proben).

Die "Stabilität" der Testvorrichtung beinhaltet Lagerstabilität, Stabilität unter physikalischen Einflüssen wie z.B. Wärme, Licht, mechanische Beanspruchung.

Die "Richtigkeit" ist ein Maß für die Abweichung des Messwertes vom richtigen Wert (manchmal als "wahrer" Wert bezeichnet) aufgrund eines systematischen Fehlers. Die Richtigkeit wird im Allgemeinen durch den Vergleich mit einem Referenz- oder Arbeitsstandard (Soll/Ist-Vergleich), den Vergleich mit einer unabhängigen, möglichst validierten Methode oder durch das sogenannte Aufstocken ("Spiken" einer Probe) bestimmt. Wenn bei bestimmten Proben keine der drei Methoden anwendbar ist, kann als Kriterium für die Richtigkeit folgendes gelten: Die Selektivität ist erwiesen, Linearität ist vorhanden, und die Kalibriergerade geht durch den Nullpunkt.

Die "Nachweisgrenze" ist die kleinste Konzentration (Menge) des Analyten in der Probe, die qualitativ noch erfasst werden kann (Ja/Nein-Entscheidung). Die "Bestimmungsgrenze" ist die kleinste Konzentration (Menge) des Analyten in der Probe, die mit gegebener Präzision und Richtigkeit quantitativ bestimmt werden kann. Das zugrunde liegende mathematische Modell und die Bestimmungsmethoden sind in der DIN 32645 beschrieben.

Die "Erfassungsgrenze" gibt die Konzentration (Menge) an, die mit einer Wahrscheinlichkeit von 50 % nachgewiesen werden kann. Somit kann die Erfassungsgrenze vereinfacht als die doppelte Nachweisgrenze angesehen werden.

### Ausführungsbeispiele

### 1. Herstellung einer Hydrogencarbonat-Testvorrichtung

Es wird eine Testlösung gemäß der folgenden Rezeptur hergestellt:

| Inhaltsstoffe | Menge |
|---|---|
| Weinsäure | 0,50 g |
| Nitrazingelb | 0,10 g |
| Bromphenolblau | 0,03 g |
| Natriumdodecylsulfat | 0,15 g |
| Nonidet P40 | 0,20 g |
| Ethanol | 15 mL |
| Wasser | 85 mL |

Ein Stück Filterpapier wird mit dieser Lösung getränkt und für 45 Sekunden bei 320 °C getrocknet. Mit Hilfe der getrockneten Test-Pads werden Teststreifen hergestellt. Die Teststreifen bestehen aus einem 5,5 x 95 mm großen PVC-Streifen als Träger auf die jeweils ein 5,5 x 5 mm großes Test-Pad mit Hilfe von Schmelzkleber befestigt ist.

### 2. Test einer Hydrogencarbonat-haltigen Testlösung

Um die Sensitivität der Testvorrichtung zu bestimmen wurden die Teststreifen in eine Dialysatlösung (Citrat/Bicarbonat) enthaltend 25; 30; 35, 40 oder 45 mEq/L Hydrogencarbonat eingetaucht. Nach 5 Sekunden wurden die Teststreifen aus der Testlösung entnommen und es wurde für 10-15 Sekunden gewartet, bis die Farbentwicklung abgeschlossen war. Die in der folgenden Tabelle dargestellten Ergebnisse zeigen, dass die Teststreifen mit steigender Hydrogencarbonat-Konzentration einen Farbübergang von grün nach blau zeigen und hierbei aufgrund der Farbe eine klare Unterscheidung zwischen den einzelnen Hydrogencarbonat-Konzentrationen von 25; 30; 35, 40 und 45 mEq/L erlauben.

| mEq/L Bicarbonat | Reaktionsfarbe |
|---|---|
| 25 | Grün |
| 30 | helles Türkis |
| 35 | Türkis |
| 40 | Türkisblau |
| 45 | Blau |

### 3. Vergleichsuntersuchung zum Einfluss von Tensiden auf die Sensitivität des Testpapiers

Ausgehend von der in Beispiel 1 aufgeführten Grundrezeptur umfassend pH-Indikator, Inertfarbstoff und Säure wurden vier Testpapiere wie folgt hergestellt:
1. ohne Tensid

| Inhaltsstoffe | Menge |
|---|---|
| Weinsäure | 0,50 g |
| Nitrazingelb | 0,10 g |
| Bromphenolblau | 0,03 g |
| Ethanol | 15 mL |
| Wasser | 85 mL |

2. nichtionisches Tensid

| Inhaltsstoffe | Menge |
|---|---|
| Weinsäure | 0,50 g |
| Nitrazingelb | 0,10 g |
| Bromphenolblau | 0,03 g |
| Nonidet P40 | 0,20 g |
| Ethanol | 15 mL |
| Wasser | 85 mL |

3. anionisches Tensid

| Inhaltsstoffe | Menge |
|---|---|
| Weinsäure | 0,50 g |
| Nitrazingelb | 0,10 g |
| Bromphenolblau | 0,03 g |
| Natriumdodecylsulfat | 0,15 g |
| Ethanol | 15 mL |
| Wasser | 85 mL |

4. Kombination aus einem nichtionischen und einem anionischen Tensid

| Inhaltsstoffe | Menge |
|---|---|
| Weinsäure | 0,50 g |
| Nitrazingelb | 0,10 g |
| Bromphenolblau | 0,03 g |
| Natriumdodecylsulfat | 0,15 g |
| Nonidet P40 | 0,20 g |
| Ethanol | 15 mL |
| Wasser | 85 mL |

Um die Sensitivität der Testpapiere zu bestimmen wurden die Teststreifen in eine Dialysatlösung (Citrat/Bicarbonat) enthaltend 25; 30; 35, 40 oder 45 mEq/L Hydrogencarbonat eingetaucht. Nach 5 Sekunden wurden die Teststreifen aus der Testlösung entnommen und es wurde für 10-15 Sekunden gewartet, bis die Farbentwicklung abgeschlossen war. Zur Beurteilung der Leistungsfähigkeit der einzelnen Testpapiere wurden die jeweiligen Farbreaktionen visuell ausgewertet und die Farbbezeichnungen für die entsprechenden Konzentrationen festgelegt.

### Ergebnisse:

In der folgenden Tabelle sind die Reaktionsfarben in Abhängigkeit von der Hydrogencarbonat-Konzentration und den Tensid-Zusätzen dargestellt:

**Tab. 1: Testergebnisse der Vergleichsmessung**

| Rezeptur | Hydrogencarbonat [mEq/L) | | | | |
|---|---|---|---|---|---|
| | **25** | **30** | **35** | **40** | **45** |
| Ohne Tensid | Grün | Grün-türkis | Türkis | Türkis-blau | Blau |
| Nichtionisches Tensid | Grün | Grün-türkis | Türkis | Türkis-blau | Blau |
| Anionisches Tensid | Grün | Grün-türkis | Türkis | Türkis-blau | Blau |
| Nichtionisches und anionisches Tensid | Helles Gelb-grün | Helles gelbtürkis | Türkis | Türkis-blau | Blau |

### Fazit:

Die spezielle Kombination aus einem nichtionischen und anionischen Tensid verschiebt die Farbreaktion im Konzentrationsbereich von 25 und 30 mEq/L Hydrogencarbonat zu gelberen Farbtönen. Somit erhöht sich die Sensitivität des Testpapiers, da eine ausgeprägtere Differenzierung zu höheren Konzentration (ab 35 mEq/L) ermöglicht wird.

Die helleren und gelberen Farbtöne im Bereich 25 und 30 mEq/L Hydrogencarbonat erzeugen, bezogen auf die gesamte Farbreihe von 25 bis 45 mEq/L, eine insgesamt besser differenzierte Farbreihe.

Die Erfindung ist zudem in der folgenden Zeichnung dargestellt und nachfolgend beschrieben.

Es zeigt:

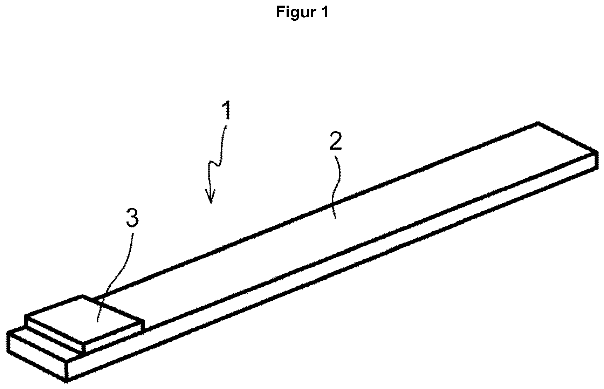

- Fig. 1: zeigt eine als Teststreifen ausgebildete Testvorrichtung mit einem Kunststoffstreifen 2 als Träger, auf das eine Trägermatrix 3 befestigt ist. Die Trägermatrix enthält die für die Bestimmung notwendigen Nachweisreagenzien, die in bevorzugter Weise eine organische Säure, einen pH-Indikator und ein Gemisch aus einem anionischen und nichtionischen Tensid sind.

### Bezugszeichenliste

- 1: Testvorrichtung
- 2: Kunststoffstäbchen
- 3: Trägermatrix

Weitere Varianten der Erfindung und ihre Ausführung ergeben sich für den Fachmann aus der vorangegangenen Offenbarung, den Figuren und den Patentansprüchen.

In den Patentansprüchen verwendete Begriffe wie "umfassen", "aufweisen", "beinhalten", "enthalten" und dergleichen schließen weitere Elemente oder Schritte nicht aus. Die Verwendung des unbestimmten Artikels schließt eine Mehrzahl nicht aus. Eine einzelne Einrichtung kann die Funktionen mehrerer in den Patentansprüchen genannten Einheiten bzw. Einrichtungen ausführen. In den Patentansprüchen angegebene Bezugszeichen sind nicht als Beschränkungen der eingesetzten Mittel und Schritte anzusehen.

## Patentansprüche

1. Testvorrichtung zur Bestimmung der Konzentration einer Base enthaltend eine Trägermatrix umfassend:
a) mindestens eine Säure, die bei Raumtemperatur fest ist;
b) mindestens einen pH Indikator mit einem Umschlagspunkt zwischen dem pH-Wert der feststofflichen Säure und der zu bestimmenden Base; und
c) ein Gemisch aus mindestens einem anionischen und mindestens einem nichtionischen Tensid.

2. Testvorrichtung gemäß Anspruch 1, **dadurch gekennzeichnet, dass** die zu bestimmende Base ausgewählt ist aus der Gruppe enthaltend Hydrogencarbonat, Natriumhydroxid, Ammoniak und Amine, wobei sie bevorzugt Hydrogencarbonat ist.

3. Testvorrichtung gemäß Anspruch 1 oder 2, **dadurch gekennzeichnet, dass** die Trägermatrix zusätzlich mindestens einen Inertfarbstoff aufweist.

4. Testvorrichtung gemäß Anspruch 3, **dadurch gekennzeichnet, dass** der mindestens eine Inertfarbstoff ausgewählt ist aus der Gruppe enthaltend Tartrazin, Neozapongelb und Nitrazingelb, wobei der Inertfarbstoff bevorzugt Nitrazingelb ist.

5. Testvorrichtung gemäß einem der vorangehenden Ansprüche, **dadurch gekennzeichnet, dass** die mindestens eine feststoffliche Säure ausgewählt ist aus der Gruppe enthaltend Zitronensäure, Bernsteinsäure, Weinsäure, Phthalsäure, Fumarsäure, Gluconsäure, Äpfelsäure, Glykolsäure, Malonsäure, Glutarsäure. Adipinsäure, Ascorbinsäure, Amidoschwefelsäure, Borsäure, Diphosphorsäure und Phosphonsäure, wobei sie bevorzugt einen pKs-Wert zwischen 2,9 und 5,6 aufweist und besonders bevorzugt Weinsäure ist.

6. Testvorrichtung gemäß einem der vorangehenden Ansprüche, **dadurch gekennzeichnet, dass** der mindestens eine pH-Indikator einen pKs-Wert von zwischen 3,8 und 7,6 aufweist und bevorzugt ausgewählt ist aus der Gruppe enthaltend Bromphenolblau, Methylorange, Tetrabromphenolblau, Kongorot, Bromkresolgrün, Lackmus und Phenolrot, und besonders bevorzugt Bromphenolblau ist.

7. Testvorrichtung gemäß einem der vorangehenden Ansprüche, **dadurch gekennzeichnet, dass** das mindestens eine nichtionische Tensid ausgewählt ist aus der Gruppe enthaltend Fettalakoholethoxylate (FAEO) wie Brij35, Fettalakoholpropoxylate (FAPO), Alkylglucoside wie Tween20, Alkylpolyglucoside (APG), Oktylphenolethoxylate, Nonidet P40 und bevorzugt Nonidet P40 ist.

8. Testvorrichtung gemäß einem der vorangehenden Ansprüche, **dadurch gekennzeichnet, dass** das mindestens eine anionische Tensid ausgewählt ist aus der Gruppe enthaltend Natriumdodecylsulfat, Ammoniumdodecylsulfat, Natriumlaurylethersulfat (SLES), Natriummyristylethersulfat, Natriumdioctylsulfosuccinat, Perfluoroctansulfonat (PFOS), Perfluorbutansulfonat, lineare Alkylbenzolsulfonate und bevorzugt Natriumdodecylsulfat ist.

9. Testvorrichtung gemäß einem der vorangehenden Ansprüche, **dadurch gekennzeichnet, dass** das molare Verhältnis zwischen dem anionischen und nichtionischen Tensid zwischen 10:1 und 1:1, bevorzugt zwischen 5:1 und 1:1, besonders bevorzugt zwischen 2:1 und 1:1, insbesondere bevorzugt zwischen 2:1 und 1,5:1 und speziell bei 1,6:1. liegt.

10. Testvorrichtung gemäß einem der vorangehenden Ansprüche, **dadurch gekennzeichnet, dass** sie als Teststreifen oder Testband ausgebildet ist oder in ein integriertes Testsystem aufnehmbar ist.

11. Testvorrichtung gemäß einem der vorangehenden Ansprüche, **dadurch gekennzeichnet, dass** die Trägermatrix ein poröses Material ist, das den Durchtritt von Flüssigkeiten erlaubt und bevorzugt ausgewählt ist aus der Gruppe enthaltend Filterpapier, Vliesstoffe, Glasfaser, poröses Polymermaterial aus Polysulfon, Polyester, Nylon, Nitrocellulose, PVDF, Polycarbonat und besonders bevorzugt Filterpapier ist.

12. Testvorrichtung gemäß einem der vorangehenden Ansprüche zur Bestimmung der Hydrogencarbonat-Konzentration, **dadurch gekennzeichnet, dass** sie ein Teststreifen mit einer Trägermatrix ist, die folgendes umfasst:
a) Weinsäure
b) Nitrazingelb
c) Bromphenolblau
d) Natriumdodecylsulfat
e) Nonidet P40

13. Testverfahren zur Bestimmung der Hydrogencarbonat-Konzentration in einer flüssigen Probe mit einer Testvorrichtung gemäß einem der vorangehenden Ansprüche, umfassend die folgenden Schritte:
a) Tränken der Trägermatrix mit der flüssigen Probe, bevorzugt durch Eintauchen in die Probe
b) Entfernen überschüssigen Probenmaterials von der Trägermatrix, bevorzugt durch Herausziehen des Teststreifens aus der Probe,
c) Optional eine Inkubation des Teststreifens für mindestens 5 Sekunden, bevorzugt bei Raumtemperatur
d) Vergleich des Farbwertes der Trägermatrix mit einem Farbstandard,
wobei die flüssige Probe bevorzugt ein Dialysat ist.

14. Verwendung eines Gemischs aus mindestens einem anionischen Tensid und mindestens einem nichtionischen Tensid zur Steigerung der Sensitivität eines Trägermatrix-vermittelten Analyseverfahrens, wobei bevorzugt ein Gemisch gemäß einem der Ansprüche 7 bis 9 verwendet wird.

## Claims

1. A test device to determine the concentration of a base containing a carrier matrix, comprising:
a) at least one acid that is solid at room temperature;
b) at least one pH indicator having an endpoint between the pH value of the solid acid and of the base that is to be determined; and
c) a mixture of at least one anionic surfactant and at least one nonionic surfactant.

2. The test device according to claim 1,
**characterized in that**
the base that is to be determined is selected from the group comprising hydrogen carbonate, sodium hydroxide, ammonia and amines, whereby it is preferably hydrogen carbonate.

3. The test device according to claim 1 or 2,
**characterized in that**
the carrier matrix additionally has at least one inert dye.

4. The test device according to claim 3,
**characterized in that**
the at least one inert dye is selected from the group comprising tartrazine, Neozapon yellow and nitrazine yellow, whereby the inert dye is preferably nitrazine yellow.

5. The test device according to one of the preceding claims,
**characterized in that**
the at least one solid acid is selected from the group comprising citric acid, succinic acid, tartaric acid, phthalic acid, fumaric acid, gluconic acid, malic acid, glycolic acid, malonic acid, glutaric acid, adipic acid, ascorbic acid, amidosulfuric acid, boric acid, diphosphoric acid and phosphonic acid, whereby it preferably has a p*K*ₐ value between 2.9 and 5.6, and it is especially preferably tartaric acid.

6. The test device according to one of the preceding claims,
**characterized in that,**
the at least one pH indicator has a p*K*ₐ value between 3.8 and 7.6 and it is preferably selected from the group comprising bromophenol blue, methyl orange, tetrabromophenol blue, Congo red, bromocresol green, litmus and phenol red, and it is especially preferably bromophenol blue.

7. The test device according to one of the preceding claims,
**characterized in that**
the at least one nonionic surfactant is selected from the group comprising fatty alcohol ethoxylates (FAEO) such as Brij 35, fatty alcohol propoxylates (FAPO), alkyl glucosides such as Tween 20, alkyl polyglucosides (APG), octylphenol ethoxcylates, Nonidet P-40, and it is preferably Nonidet P-40.

8. The test device according to one of the preceding claims,
**characterized in that,**
the at least one nonionic surfactant is selected from the group comprising sodium dodecyl sulfate, ammonium dodecyl sulfate, sodium lauryl ether sulfate (SLES), sodium myristyl ether sulfate, sodium dioctyl sulfosuccinate, prefluorooctane sulfonate (PFOS), perfluorobutane sulfonate, linear alkylbenzene sulfonates, and it is preferably sodium dodecyl sulfate.

9. The test device according to one of the preceding claims,
**characterized in that**
the molar ratio between the anionic surfactant and the nonionic surfactant is between 10:1 and 1:1, preferably between 5:1 and 1:1, especially preferably between 2:1 and 1:1, particularly preferred between 2:1 and 1.5:1 and especially 1.6:1.

10. The test device according to one of the preceding claims,
**characterized in that**
it is configured as a test strip or test tape, or else it can be incorporated into an integrated test system.

11. The test device according to one of the preceding claims,
**characterized in that**
the carrier matrix is a porous material that allows the passage of liquids and that is preferably selected from the group comprising filter paper, nonwovens, glass fibers, porous polymer material consisting of polysulfone, polyester, nylon, nitrocellulose, PVDF, polycarbonate, and it is especially preferably filter paper.

12. The test device according to one of the preceding claims to determine the concentration of hydrogen carbonate,
**characterized in that**
it is a test strip with a carrier matrix that encompasses the following:
a) tartaric acid
b) nitrazine yellow
c) bromophenol blue
d) sodium dodecyl sulfate
e) Nonidet P-40

13. A test method to determine the concentration of hydrogen carbonate in a liquid sample, using a test device according to one of the preceding claims, encompassing the following steps:
a) soaking the carrier matrix with the liquid sample, preferably by dipping it into the sample;
b) removing excess sample material from the carrier matrix, preferably by lifting the test strip out of the sample;
c) optionally incubating the test strip for at least 5 seconds, preferably at room temperature;
d) comparing the color value of the carrier matrix to a color standard,
whereby the liquid sample is preferably a dialysate.

14. Use of a mixture consisting of at least one anionic surfactant and at least one nonionic surfactant in order to increase the sensitivity of a carrier matrix-mediated analytical method, whereby preferably a mixture according to one of claims 7 to 9 is used.

## Revendications

1. Dispositif d'essai pour déterminer la concentration d'une base contenant une matrice de support comprenant :
a) au moins un acide solide à température ambiante ;
b) au moins un indicateur de pH avec un point de transition entre le pH de l'acide solide et la base à déterminer ; et
c) un mélange d'au moins un tensioactif anionique et au moins un tensioactif non ionique.

2. Dispositif d'essai selon la revendication 1, **caractérisé en ce que** la base à déterminer est choisie dans le groupe comprenant le carbonate d'hydrogène, l'hydroxyde de sodium, l'ammoniac et les amines, et étant de préférence du carbonate d'hydrogène.

3. Dispositif d'essai selon la revendication 1 ou 2, **caractérisé en ce que** la matrice de support comporte en outre au moins un colorant inerte.

4. Dispositif de test selon la revendication 3, **caractérisé en ce que** l'au moins un colorant inerte est choisi dans le groupe comprenant la tartrazine, le jaune Néozapon et le jaune de nitrazine, le colorant inerte étant de préférence du jaune de nitrazine.

5. Dispositif d'essai selon l'une des revendications précédentes, **caractérisé en ce que** l'au moins un acide solide est choisi dans le groupe comprenant l'acide citrique, l'acide succinique, l'acide tartrique, l'acide phtalique, l'acide fumarique, l'acide gluconique, l'acide malique, l'acide glycolique, l'acide malonique, l'acide glutarique, l'acide adipique, l'acide ascorbique, l'acide amidosulfurique, l'acide borique, l'acide diphosphorique et l'acide phosphonique, ledit acide ayant une valeur pKs comprise entre 2,9 et 5,6 et étant, de manière particulièrement préférentielle, de l'acide tartrique.

6. Dispositif de test selon l'une des revendications précédentes, **caractérisé en ce que** l'au moins un indicateur de pH a une valeur pKs comprise entre 3,8 et 7,6 et est de préférence choisi dans le groupe comprenant le bleu de bromophénol, le bleu de méthyle orange, le bleu de tétrabromophénol, le rouge Congo, le vert de bromocrésol, le litmus et le rouge de phénol, et, de manière particulièrement préférentielle, le bleu de bromophénol.

7. Dispositif de test selon l'une des revendications précédentes, **caractérisé en ce que** l'au moins un tensioactif non ionique est choisi dans le groupe comprenant les éthoxylates d'alcool gras (FAEO) tels que Brij35, les propoxylates d'alcool gras (FAPO), les glucosides d'alkyle tels que Tween20, les polyglucosides d'alkyle (APG), les octylphénoléthoxylates, le Nonidet P40, et est de préférence du Nonidet P40.

8. Dispositif d'essai selon l'une des revendications précédentes, **caractérisé en ce que** l'au moins un agent tensioactif anionique est choisi dans le groupe comprenant le dodécylsulfate de sodium, le dodécylsulfate d'ammonium, le lauryl éther sulfate de sodium (SLES), myristyléther sulfate de sodium, dioctylsulfosuccinate de sodium, le perfluorooctane sulfonate (PFOS), le perfluorobutane sulfonate, les sulfonates linéaires d'alkylbenzène, et est de préférence du dodécylsulfate de sodium.

9. Dispositif de test selon l'une des revendications précédentes, **caractérisé en ce que** le rapport molaire entre le tensioactif anionique et le tensioactif non ionique est compris entre 10:1 et 1:1, de préférence entre 5:1 et 1:1, de manière particulièrement préférentielle 2:1 et 1:1, de manière plus particulièrement préférentielle entre 2:1 et 1,5:1, et est spécialement de 1,6 :1.

10. Dispositif d'essai selon l'une des revendications précédentes, **caractérisé en ce qu'**il est conçu sous forme de bandelette-test ou de bande-test ou peut être logé dans un système d'essai intégré.

11. Dispositif d'essai selon l'une des revendications précédentes, **caractérisé en ce que** la matrice de support est un matériau poreux qui permet le passage de liquides et est de préférence choisi dans le groupe comprenant le papier-filtre, les non-tissés, la fibre de verre, le matériau polymère poreux de polysulfone, le polyester, le nylon, la nitrocellulose, le PVDF, le polycarbonate et est, de manière particulièrement préférentielle, du papier-filtre.

12. Dispositif d'essai selon l'une des revendications précédentes pour déterminer la concentration de carbonate d'hydrogène, **caractérisé en ce qu'**il s'agit d'une bandelette d'essai ayant une matrice de support, comprenant ce qui suit :
a) acide tartrique
b) jaune de nitrazine
c) bleu de bromophénol
d) dodécylsulfate de sodium
e) Nonidet P40.

13. Procédé d'essai pour déterminer la concentration de carbonate d'hydrogène dans un échantillon liquide à l'aide d'un dispositif d'essai selon l'une des revendications précédentes, comprenant les étapes suivantes :
a) imprégnation de la matrice de support avec l'échantillon liquide, préférentiellement par immersion dans l'échantillon ;
b) retrait de la matière d'échantillon excédentaire de la matrice de support, de préférence en retirant la bandelette d'essai de l'échantillon ;
c) incubation optionnelle de la bandelette d'essai pendant au moins 5 secondes, de préférence à température ambiante ;
d) comparaison de la valeur de couleur de la matrice de support avec un étalon de couleur, l'échantillon liquide étant de préférence un dialysat.

14. Utilisation d'un mélange d'au moins un tensioactif anionique et d'au moins un tensioactif non ionique pour augmenter la sensibilité d'un procédé d'analyse à matrice de support, de préférence en utilisant un mélange selon l'une des revendications 7 à 9 étant utilisé préférentiellement.
